# EUROPÄISCHE PATENTSCHRIFT

(11) **EP 2 298 204 B1**
(45) Veröffentlichungstag und Bekanntmachung des Hinweises auf die Patenterteilung: **23.07.2014**
(21) Anmeldenummer: 10176528.7
(22) Anmeldetag: 14.09.2010
(51) Int. Cl.: A61B 18/14, A61B 18/00

(54) **Medizinisches Instrument für die bipolare Elektrochirurgie**
Medical instrument for bipolar electrosurgery
Instrument médical pour l'électrochirurgie bipolaire

(30) Priorität: 21.09.2009 DE 102009047912
(43) Veröffentlichungstag der Anmeldung: 23.03.2011
(73) Patentinhaber: Karl Storz GmbH & Co. KG, 78532 Tuttlingen (DE)
(72) Erfinder: Blocher, Martin, 78532, Tuttlingen (DE); Schneider, Sven, 78532, Tuttlingen (DE); Scarfi, Andrea, 73760, Ostfildern (DE)
(74) Vertreter: Witte, Weller & Partner Patentanwälte mbB

(56) Entgegenhaltungen:
- WO-A1-97/40759
- US-A- 5 290 286
- US-A1- 2006 020 265
- US-B1- 6 190 384

## Beschreibung

Die Erfindung betrifft ein medizinisches Instrument für die bipolare Elektrochirurgie, mit einem Außenschaft, an dessen distalem Ende eine elektrisch leitende Schneide angeordnet ist, die gegenüber dem Außenschaft elektrisch isoliert ist, und mit einem im Außenschaft aufgenommenen und in diesem axial hin- und herbewegbaren Arbeitseinsatz, an dessen distalem Ende ein Haken angeordnet ist, der eine Schneidekante aufweist, die mit der Schneide des Außenschaftes schneidend zusammenwirkt, dadurch gekennzeichnet, dass am Arbeitseinsatz eine erste Stromleitung vorhanden ist, die zumindest die Schneidekante des Hakens elektrisch versorgt, und dass am Arbeitseinsatz eine zweite Stromleitung vorhanden ist, die distal in einem äußeren Kontakt mündet, der mit der elektrisch leitenden Schneide des Außenschaftes in Verbindung steht.

Ein derartiges Instrument ist aus der US-A-5 290 286 bekannt.

Ein solches Instrument wird bspw. zur Durchführung endoskopisch gestützter Eingriffe im menschlichen oder tierischen Körper im Rahmen der minimal-invasiven Chirurgie verwendet.

Mit einem elektrochirurgischen medizinischen Instrument kann biologisches Gewebe unter dem Einfluss von Hochfrequenzstrom koaguliert und/oder geschnitten werden.

Bei der bipolaren Elektrochirurgie wird das Gewebe zwischen die beiden mit Hochfrequenzstrom beaufschlagten Pole gebracht und dort zunächst koaguliert. Dies dient bspw. dazu, Gefäße zu verschließen. Soll noch ein Schnitt durchgeführt werden, können die beiden Pole weiter beaufschlagt werden oder näher zusammengebracht werden, so dass das Gewebe nicht nur koaguliert, sondern auch durchtrennt wird.

Dazu sind nun unterschiedliche Ausgestaltungen an medizinischen Instrumenten bekannt.

Aus der US-A-5 190 541 ist ein bipolares Instrument bekannt, bei dem in einem Schaft ein erstes stromführendes Element vorhanden ist, dessen distales Ende einen etwa rechtwinklig zu der Längsachse abgewinkelten Haken aufweist. Das stirnseitige Ende des Hakens ist als gezackte Schneidekante ausgebildet. Das proximale Ende des Hakens kann mit einem weiteren im Schaft angeordneten stromführenden Schneidemesser dahingehend zusammenwirken, dass diese beiden Elemente aufeinander zu bewegt werden.

Beidseitig des mittigen stromführenden Schneidemessers sind noch gleichpolige Koagulationsstangen angeordnet. Anhand dieser Koagulationsstangen kann bspw. ein Gefäß an zwei voneinander beabstandeten Stellen im Zusammenwirken mit dem ersten stromführenden Element zunächst koaguliert werden. Anschließend kann durch Vortreiben des mittigen Schneidemessers das Gefäß noch zwischen den Koagulationsstellen aufgetrennt werden.

Nachteilig an dieser Ausgestaltung ist, dass im Inneren des Schafts insgesamt vier stromführende einzelne Bauelemente aufgenommen sind.

Dies ist nicht nur ein komplizierter Aufbau, ein solches Gerät ist auch schwierig zu reinigen.

Aus der US 2003/0040744 A1 ist ein bipolares Koagulationsgerät bekannt, das insgesamt drei Elektroden aufweist.

Ein Haken, der über einen Pol beaufschlagt ist, ist beidseitig von zwei stromführenden Hälften umgeben, zwischen denen der Haken bewegbar ist.

Ähnlich wie beim zuvor beschriebenen Stand der Technik, dienen die beiden beidseits des Hakens angeordneten Hälften zum Koagulieren, soll ein Schneidevorgang durchgeführt werden, werden alle drei Bauelemente aktiviert.

Nachteilig ist hier ebenfalls ein sehr komplizierter Aufbau, der im Inneren drei Leitungen erfordert und somit schwierig zu reinigen ist.

Aus der eingangs erwähnten US-A-5 290 286 ist ein bipolares Koagulationsinstrument bekannt, bei dem an einem distalen Abschlussstopfen des Rohrschaftes eine stationäre Elektrode angebracht ist, deren distales Ende als Schneide ausgebildet ist. Im Rohrschaft ist ferner eine bewegliche Elektrode aufgenommen, die über eine Handhabe im Rohrschaft hin- und herbewegt werden kann.

Das distale Ende der beweglichen Elektrode ist rechtwinklig abgebogen, so dass sich ein hakenartiges Gebilde ergibt. Diese bewegliche Elektrode kann auf eine Schneide der stationären Elektrode zu bewegt werden, bis beide aneinanderstoßen, so dass neben einer Koagulation auch ein Trennvorgang durchgeführt werden kann.

Dieses Gerät ist aufgrund der stationär ausgebildeten Elektrode, durch die die bewegliche Elektrode hindurchgeführt ist, schwierig zu zerlegen und zu reinigen.

Aus der US 5,484,435 ist ein bipolares Instrument bekannt, in dem in einem Rohrschaft zwei Stromzuführungen zu zwei Elektroden geführt sind. Eine mittige Elektrode ist von einer äußeren U-förmigen Elektrode umgeben.

Die sehr filigran aufgebauten Elektroden liegen sehr eng beieinander, so dass Stromüberschläge nicht ausgeschlossen werden können. Ferner sind diese Elektroden aufgrund des filigranen und komplizierten Aufbaus nur sehr schwierig zu reinigen.

Aus der DE 20 2004 010 780 U1 ist ein bipolares Rohrschaftinstrument bekannt, bei dem am distalen Ende zwei bewegliche Maulteile vorgesehen sind. Beim Öffnen und Schließen der Maulteile wird ein drahtförmiges stromzuführendes Element permanent gebogen und wieder gestreckt. Zur Behebung dieses Problems wird an dem beweglichen Maulteil ein Schleifkontakt vorgesehen, der den Strom an der Schwenkachse des beweglichen Maulteils abgreift.

Es ist Aufgabe der vorliegenden Erfindung, ein medizinisches Instrument für die bipolare Elektrochirurgie zu schaffen, das einfach zerlegbar und reinigbar ist, und mit dem die beiden Vorgänge Koagulieren und Schneiden sicher und einfach durchführbar sind.

Erfindungsgemäß wird die Aufgabe durch ein medizinisches Instrument für die bipolare Elektrochirurgie dadurch gelöst, dass der Arbeitseinsatz Komplett vom Aussenschaft abgezogen und wieder in den Aussenschaft eingeschoben werden kann und dass die Stromversorgung der Schneide durch den im Außenschaft aufgenommenen bewegbaren Arbeitseinsatz erfolgt, wobei der Arbeitseinsatz derart ausgestaltet ist, dass der Kontakt der zweiten Stromleitung mit der elektrisch leitenden Schneide in Berührung tritt, wenn der Arbeitseinsatz eingeschoben ist.

Diese Maßnahmen haben die folgenden Vorteile.

Der innere Arbeitseinsatz stellt ein kompaktes Bauteil dar, das die komplette Stromversorgung beinhaltet und bewirkt. Dieser Arbeitseinsatz kann komplett vom Außenschaft abgezogen werden, bspw. um nach einem Einsatz gereinigt zu werden, wobei nach einem Koagulieren dies insbesondere an den Polen notwendig ist, da dort koaguliertes und ggf. verbranntes Gewebe anhaftet. Nach dem Reinigen kann die Vorrichtung einfach dadurch montiert werden, dass der komplette Arbeitseinsatz wieder in den Außenschaft eingeschoben wird.

Die Maßnahme, dass am Außenschaft eine elektrisch leitende Schneide angeordnet ist, die gegenüber dem Außenschaft elektrisch isoliert ist, hat den Vorteil, dass der Außenschaft selbst nicht zur Stromleitung herangezogen wird. Somit können auch allfällige Beschädigungen nicht zu unerwünschten Stromschlägen, insbesondere für die Handhabungsperson führen. Dennoch ist ein leitender Pol vorgesehen, nämlich die elektrisch leitende Schneide am distalen Ende.

Die Stromversorgung dieser elektrisch leitenden Schneide am Außenschaft erfolgt durch den Arbeitseinsatz, der so ausgestaltet ist, dass der Kontakt der zweiten Stromleitung mit der elektrisch leitenden Schneide in Berührung tritt, wenn der Arbeitseinsatz eingeschoben ist.

Diese elektrisch leitende Schneide kann als ein Pol beim Koagulieren herangezogen werden und kann, auch ohne Strombeaufschlagung, als mechanische Schneide zum Durchtrennen des Gewebes dienen.

Diese Ausgestaltung eröffnet auch die Möglichkeit, sowohl die elektrisch leitende Schneide am distalen Ende des Außenschaftes als auch den Haken relativ stabil auszubilden, so dass diese beiden Teile auch als rein mechanische Schneidewerkzeuge dienen können. Dieses Schneiden erfolgt dadurch, dass der Haken bzw. dessen Schneidekante an der Schneide am distalen Ende des Außenschaftes vorbeigeführt wird und dabei das Gewebe, wie bei einem Scherenschnitt, exakt abtrennt.

Somit kann zum Koagulieren das Gewebe durch den Haken ergriffen und an die Schneide am distalen Ende des Außenschaftes herangeführt werden. Bei Strombeaufschlagung kann zunächst ein Koaguliervorgang durchgeführt werden.

Je nach Wunsch, kann dann ein Auftrennen durchgeführt werden, sei es unter Strombeaufschlagung oder als rein mechanischer Schneidevorgang.

Dieser kann besonders exakt und gerichtet durchgeführt werden.

Somit steht nicht nur ein mechanisch stabiler und robuster Zusammenbau zur Verfügung, der einfach zerlegt und gereinigt werden kann, sondern es besteht eine variable Konstruktion, um einen Koagulationsvorgang und/oder elektrisch unterstützte oder rein mechanische Schneidevorgänge sicher durchzuführen.

Somit wird die Aufgabe vollkommen gelöst.

In einer weiteren Ausgestaltung der Erfindung ist die erste Stromleitung isoliert im Inneren des Arbeitseinsatzes zur Schneidekante des Hakens geführt.

Diese Maßnahme hat den Vorteil, dass diese Stromleitung vor äußeren mechanischen Einflüssen geschützt zum Haken geführt werden kann.

In einer weiteren Ausgestaltung der Erfindung ist die äußere Seite des Hakens wenigstens teilweise elektrisch isoliert.

Dadurch kann die Größe der Elektrodenfläche variiert werden.

Der Haken kann rein mechanisch zum Ergreifen des Gewebes herangezogen werden. Eine Strombeaufschlagung erfolgt nur jeweils im nicht isolierten Bereich, so dass an genau definierten Stellen der Koagulationsvorgang durchgeführt werden kann.

In einer weiteren Ausgestaltung der Erfindung ist die zweite Stromleitung isoliert im Inneren des Arbeitseinsatzes zum Kontakt geführt.

Diese Maßnahme hat den Vorteil, dass auch die zweite Stromleitung vor äußeren Einflüssen geschützt bis zu der Kontaktstelle mit der Schneide am Außenschaft geführt ist.

In einer weiteren Ausgestaltung der Erfindung ist der Kontakt als Schleifkontakt ausgebildet, der beim Hin- und Herbewegen des Arbeitseinsatzes im Außenschaft über die Schneide fährt.

Diese Maßnahme hat den Vorteil, dass über den Hub bzw. Weg des Arbeitseinsatzes, über den er im Außenschaft hin- und herbewegt wird, dauernd sichergestellt ist, dass ein elektrischer Kontakt zwischen dem Kontakt der zweiten Stromleitung am inneren Arbeitseinsatz und der Schneide am Außenschaft besteht. Dies erhöht erheblich die Betriebssicherheit.

In einer weiteren Ausgestaltung der Erfindung ist die Schneide proximalseitig mit einem umfänglich umlaufenden Abschnitt verbunden, den der Kontakt kontaktiert.

Diese Maßnahme hat den Vorteil, dass vollumfänglich eine Kontaktfläche für den Kontakt zur Verfügung steht. Der Rohrabschnitt stellt außerdem eine Verstärkung dar.

In einer weiteren Ausgestaltung der Erfindung ist der Kontakt als seitlich vom Arbeitsteil vorspringender Bügel ausgebildet.

Diese Maßnahme hat den Vorteil, dass der innere Arbeitseinsatz selbst in einem gewissen radialen Abstand von der Schneide angeordnet sein kann, und lediglich der vorspringende Bügel den elektrisch leitenden Kontakt herstellt.

In einer weiteren Ausgestaltung der Erfindung ist der Bügel seitlich so ausgebogen, dass er unter einem Anpressdruck über den Abschnitt der Schneide läuft.

Diese Maßnahme hat den Vorteil, dass der elektrische Kontakt besonders betriebssicher ist. Durch den Anpressdruck werden auch allfällige Kontaminationen, die an der Kontaktstelle anhaften könnten, vom Bügel weggeschoben oder abgerieben. Es können auch Toleranzen und Unebenheiten der Bauteile ausgeglichen werden.

In einer weiteren Ausgestaltung der Erfindung ist die Schneide als vom distalen Ende des Außenschafts vorspringendes Einsatzstück ausgebildet.

Diese Maßnahme hat den Vorteil, dass das Einsatzstück unabhängig von der Geometrie des Außenschaftes so ausgestaltet werden kann, dass ein optimales Zusammenwirken mit einem entsprechend ausgebildeten Haken möglich ist.

In anderen Worten ausgedrückt, können Haken und Einsatzstück jeweils optimal aufeinander abgestimmt werden, damit sowohl der Koagulationsvorgang als auch ein gewünschter Schneidevorgang exakt abläuft.

Dies ermöglicht auch bei schlanken Geräten, diese beiden Bauelemente, nämlich Einsatzstück und Haken, relativ kompakt auszubilden, so dass insbesondere mechanische Schnitte exakt durchgeführt werden können.

In einer weiteren Ausgestaltung der Erfindung weist die Schneide distalseitig eine Schneidekante auf, an der die Schneidekante des Hakens vorbei bewegbar ist.

Diese Maßnahme hat den erheblichen Vorteil, dass der Schneidevorgang in Art eines scherenartigen Schneidevorganges abläuft, so dass besonders saubere und glatte Schnitte durchgeführt werden können. Bei dem eingangs genannten Stand der Technik waren beim Schneidevorgang Schneidekanten vorhanden, die direkt aufeinandertreffen, so dass zwangsläufig, zumindest längs der Schneidelinien, Geweberückstände verblieben, so dass letztendlich im Wesentlichen nur ein elektrisch unterstütztes Schneiden und kein mechanisches Schneiden im Sinne eines Scherenschnittes möglich war.

In einer weiteren Ausgestaltung der Erfindung verläuft die Schneidekante der Schneide unter einem Winkel zur Schneidekante des Hakens.

Diese Maßnahme hat den Vorteil, dass dadurch ein besonders exakter Schnitt möglich ist.

In einer weiteren Ausgestaltung der Erfindung ist die Schneidekante des Hakens proximalseitig am Haken angeordnet.

Diese Maßnahme hat den Vorteil, dass beim Einziehen des Hakens in den Außenschaft dessen Schneide an der feststehenden Schneide am Außenschaft vorbeiläuft, wodurch ein genau kontrollierbarer sicherer glatter Schnitt möglich ist.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand eines ausgewählten Ausführungsbeispiels in Zusammenhang mit den beiliegenden Zeichnungen näher beschrieben und erläutert. Es zeigen:
- Fig. 1: eine perspektivische Ansicht des medizinischen Instrumentes von distal nach proximal mit eingezogenem Arbeitseinsatz;
- Fig. 2: eine stark vergrößerte Darstellung des in Fig. 1 mit einem Kreis umrundeten distalen Endbereiches mit ausgefahrenem Arbeitseinsatz;
- Fig. 3: eine Seitenansicht des distalen Endbereiches des Arbeitseinsatzes;
- Fig. 4: eine um 90° um dessen Längsachse verdrehte Seitenansicht;
- Fig. 5: eine gegenüber der Fig. 3 um 180° verdrehte Seitenansicht des Arbeitseinsat- zes;
- Fig. 6: eine gegenüber der Fig. 5 um weitere 90° verdrehte Seitenansicht des Ar- beitseinsatzes;
- Fig. 7: einen Längsschnitt des Außenschaftes des medizinischen Instrumentes von Fig. 1;
- Fig.8: eine vergrößerte Darstellung des in Fig. 7 mit einem Kreis umrundeten distalen Endbereiches des Außenschaftes;
- Fig. 9: einen Schnitt längs der Linie IX-IX in Fig. 8;
- Fig. 10: eine Seitenansicht des medizinischen Instrumentes von Fig. 1 mit ausgefah- renem Arbeitseinsatz;
- Fig. 11: einen stark vergrößerten Längsschnitt des in Fig. 10 mit einem Kreis umrun- deten distalen Endbereiches des Außenschaftes, und
- Fig. 12: einen Schnitt längs der Linie XII-XII in Fig. 11.

Ein in den Figuren dargestelltes medizinisches Instrument ist in seiner Gesamtheit mit der Bezugsziffer 10 bezeichnet.

Das medizinische Instrument 10 dient der bipolaren Elektrochirurgie und weist einen rohrförmigen Außenschaft 12 auf, dessen distales Ende 14 mit einer Schneide 16 versehen ist.

Am proximalen Ende 18 ist der Außenschaft 12 mit einer Handhabe 20 verbunden, die ein feststehendes Griffteil 22 und ein bewegliches Griffteil 24 aufweist. Proximal stehen von der Handhabe 20 zwei HF-Anschlüsse 26 und 27 vor.

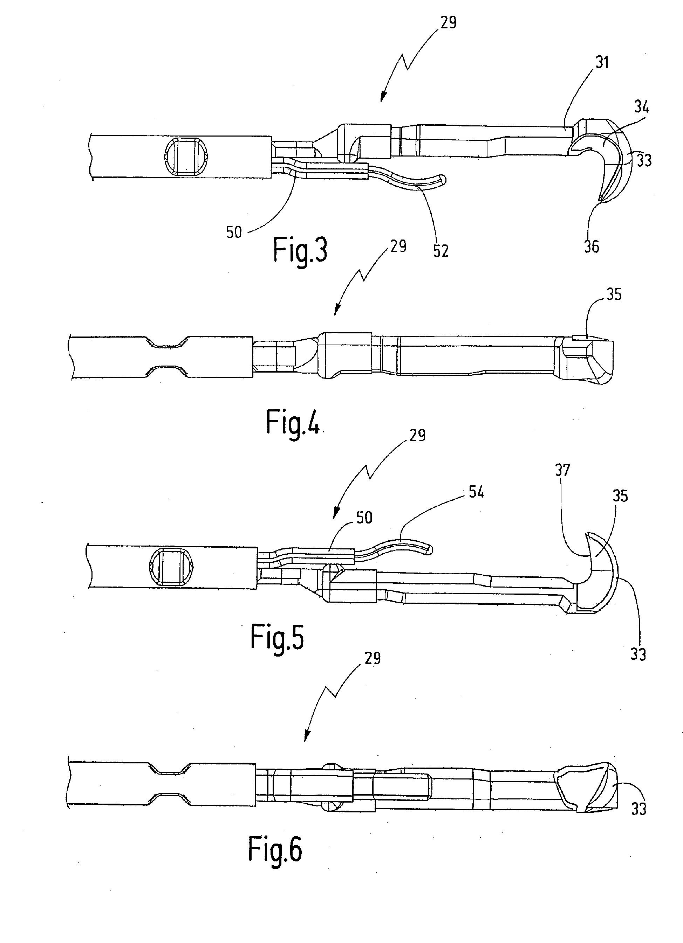

Im Inneren des Außenschaftes 12 ist ein Arbeitseinsatz 29 aufgenommen, der insbesondere in den Figuren 3 bis 6 näher dargestellt ist.

Der Arbeitseinsatz 29 weist an seinem distalen Ende 31 einen Haken 33 auf. Der Haken 33 selbst ist als eine Art kalottenförmiger Körper 34 ausgebildet, der an seiner äußeren Seite in eine Spitze 36 ausläuft. Auf der Seite, die der Schneide 16 am Außenschaft 12 zugewandt ist, weist der Haken 33 ebenfalls eine Schneide 35 auf, deren Schneidekante 37 nach proximal gerichtet ist (siehe Fig. 5).

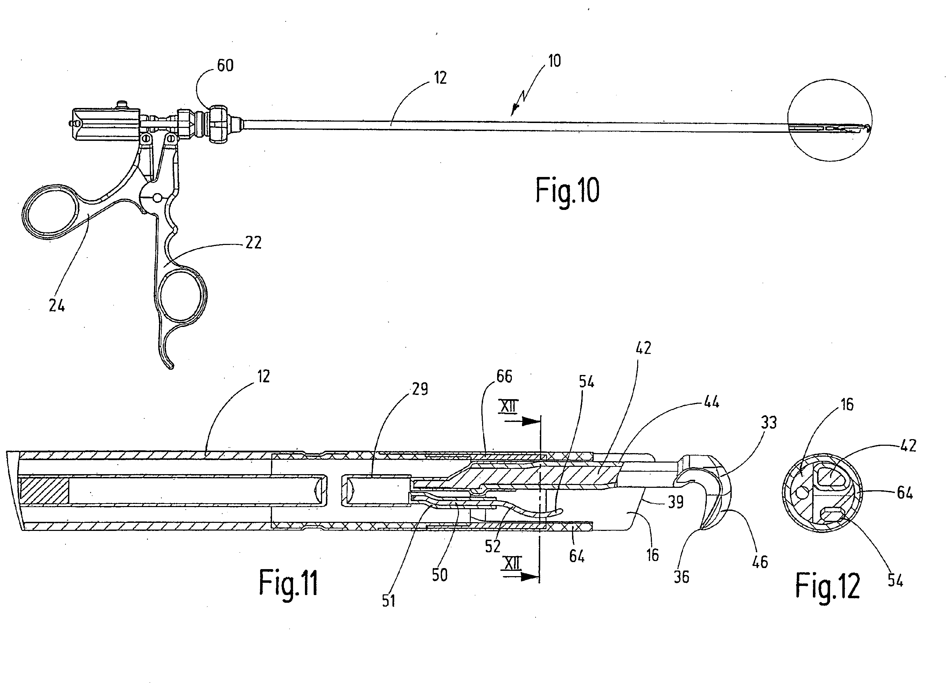

Wie insbesondere aus Fig. 11 zu erkennen, ist der Haken 33 mit einer ersten Stromleitung 42 verbunden, die im Inneren des Arbeitseinsatzes 29 bis zu dessen proximalem Ende reicht und dort mit einem der HF-Anschlüsse verbunden ist. Die erste Stromleitung 42 ist mit einer isolierenden Umhüllung 44 umgeben.

Auch der Haken 33 selbst bzw. Teilbereiche dessen äußerer Seite sind mit einer Isolierung 46, z.B. aus Keramik, versehen. Allerdings ist der Bereich der Schneide 35 nicht isoliert und somit stromführend.

Diese Stelle stellt somit einen ersten Pol dar, und zwar den sog. aktiven Pol.

Insbesondere aus der Schnittdarstellung von Fig. 11 ist weiter zu erkennen, dass im Arbeitseinsatz 29 noch eine zweite Stromleitung 50 vorhanden ist, die proximalseitig in einem Kontakt 52 endet. Auch die Stromleitung 50 ist mit einer isolierenden Umhüllung 51 umgeben.

Der Kontakt 52 ist als ein Schleifkontakt ausgebildet und hat die Form eines radial nach außen gebogenen Bügels 54. Die Ausformung ist so, wie das insbesondere aus Fig. 12 zu erkennen ist, dass der Bügel 54 dauernd elektrisch leitend mit einem Rohrabschnitt 64 in Verbindung steht, der wiederum leitend mit der Schneide 16 verbunden ist.

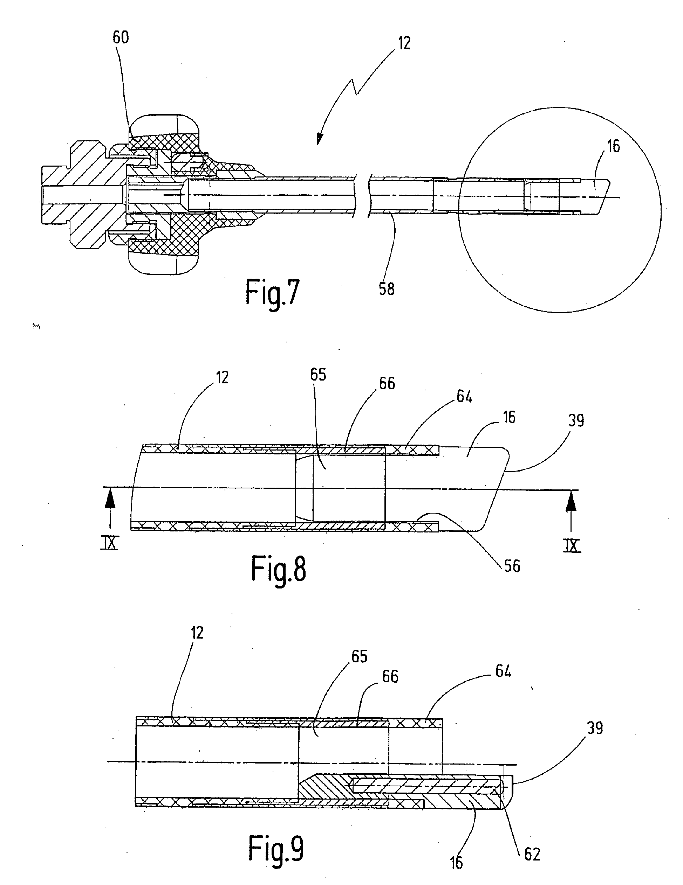

Wie insbesondere aus Figuren 8 und 9 ersichtlich, sind Rohrabschnitt 64 und Schneide 16 gegenüber dem Außenschaft 12 durch ein isolierendes Zwischenstück 66 elektrisch isoliert. Die Schneide 16 ist als ein grob plattenförmiges Einsatzstück 56 ausgeformt, das über einen Stift 62 am Rohrabschnitt 64 fixiert ist.

Ein proximales Ende der Schneide 16 ist als ein umfänglich umlaufender Abschnitt 65 ausgebildet, auf dem der Kontakt 52 bzw. der Bügel 54 läuft.

Auch die zweite Stromleitung 50 ist durch den Arbeitseinsatz 29 bis zu dessen proximalen Ende geführt und dort mit einem der beiden HF-Anschlüsse verbunden.

Das proximale Ende 18 des Außenschaftes 12 ist über eine Kupplung 60 mit der Handhabe 20 verbunden, durch die es möglich ist, den Außenschaft 12 um dessen Längsachse zu drehen, wie das an sich bekannt ist.

Das hier nicht dargestellte proximale Ende des Arbeitseinsatzes 20 ist ebenfalls in an sich bekannter Weise mit dem beweglichen Griffteil 24 der Handhabe gekoppelt, so dass ein Verschwenken des verschwenkbaren Griffteiles 24 ein Hin- und Herbewegen des Arbeitseinsatzes 29 im Außenschaft 12 bewirkt.

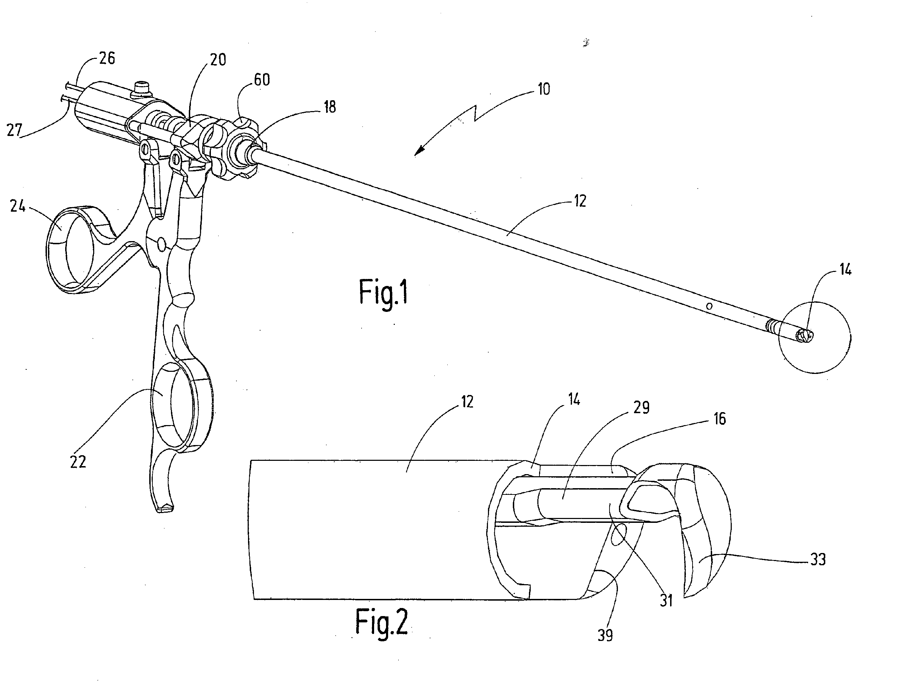

Dabei wird der Arbeitseinsatz 29 zwischen einer Position, wie sie in Fig. 11 gezeigt ist, also bei einem maximal ausgefahrenen Arbeitseinsatz 29, bis zu einer in Fig. 1 dargestellten Position mit maximal eingefahrenem Arbeitseinsatz 29 bewegt.

In der in Fig. 11 dargestellten ausgefahrenen Stellung kann von dem Haken 33, bspw. durch Einstechen seiner Spitze 36, Gewebe als solches ergriffen und durch Einziehen des Arbeitseinsatzes 29 in den Außenschaft 12 in Richtung auf die Schneide 16 bewegt werden, die den zweiten Pol, den sog. passiven Pol des bipolaren medizinischen Instrumentes darstellt. Werden nunmehr die beiden Pole, also Haken 33 und Schneide 16, mit Hochfrequenzstrom beaufschlagt, wird das dazwischen gehaltene Gewebe koaguliert. Soll das Gewebe auch noch aufgetrennt werden, wird der Arbeitseinsatz 29 weiter in das Innere des Außenschaftes 12 hineinbewegt, so dass dann dessen insbesondere aus Fig. 5 ersichtliche Schneidekante 37 an der insbesondere aus Fig. 11 ersichtlichen Schneidekante 39 der Schneide 16 vorbeiläuft.

Es ist ersichtlich, dass diese Schneidekanten 37 und 39 unter einem Winkel zueinanderstehen, so dass letztendlich eine scherenartige Schnittbewegung resultiert, die für ein besonders scharfes und glattes Auftrennen des koagulierten Gewebes sorgt. Dieser Schneidevorgang kann nun mit oder ohne Strombeaufschlagung stattfinden.

Da aufgrund der zuvor beschriebenen Konstruktion ein scherenartiger Schnitt erfolgt, kann es wünschenswert sein, solche Schnitte immer ohne Strombeaufschlagung durchzuführen. Dann können Maßnahmen vorgesehen sein, die bei einem Annähern der Schneidekante 37 an die Schneidekante 39, bspw. durch einen Positionssensor oder dergleichen, dafür sorgen, dass ein Stromfluss ausgeschlossen ist.

Dies kann bspw. schaltungstechnisch an der Handhabe stattfinden.

Nach einem Einsatz kann die Handhabe 20 vom proximalen Ende des Außenschaftes 12 gelöst werden und der komplette einzige Arbeitseinsatz 29 kann vom Außenschaft 12 abgezogen werden. In diesem zerlegten Zustand kann insbesondere der Haken 33 gut gereinigt werden, selbst anhaftendes Gewebe ist dabei einfach abzulösen, wobei das noch dadurch erleichtert wird, dass lediglich die von der Außenseite gut zugängliche Schneide 35 des Hakens 33 mit Strom beaufschlagt wird, sich also nur dort eventuell Koagulationsrückstände absetzen konnten.

Gleiches gilt für die Schneide 16, die vom Außenschaft 12 nach distal vorsteht, auch diese ist gut zu reinigen, denn deren Stirnseite sowie deren Innenseite ist für einen Reinigungsvorgang gut zugänglich.

Somit sind nicht nur einfacher Aufbau und eine robuste Bauweise mit sicherer Funktion erzielt, sondern das Instrument kann auch einfach zerlegt, gereinigt und wieder montiert werden.

## Patentansprüche

1. Medizinisches Instrument für die bipolare Elektrochirurgie,
mit einem Außenschaft (12), an dessen distalem Ende (14) eine elektrisch leitende Schneide (16) angeordnet ist, die gegenüber dem Außenschaft (12) elektrisch isoliert ist, und
mit einem im Außenschaft (12) aufgenommenen und in diesem axial hin- und herbewegbaren Arbeitseinsatz (29), an dessen distalem Ende (31) ein Haken (33) angeordnet ist, der eine Schneidekante (37) aufweist, die mit der Schneide (16) des Außenschaftes (12) schneidend zusammenwirkt,
wobei am Arbeitseinsatz (29) eine erste Stromleitung (42) vorhanden ist, die zumindest die Schneidekante (37) des Hakens (33) elektrisch versorgt, und dass am Arbeitseinsatz (29) eine zweite Stromleitung (50) vorhanden ist, die distal in einem äußeren Kontakt (52) mündet, der mit der elektrisch leitenden Schneide (16) des Außenschaftes (12) in Verbindung steht,
**dadurch gekennzeichnet, dass** der Arbeitseinsatz komplett vom Aussenschaft (12) abgezogen und wieder in den Aussenschaft (12) eingeschoben werden kann und dass die Stromversorgung der Schneide (16) durch den im Außenschaft aufgenommenen bewegbaren Arbeitseinsatz (29) erfolgt,
wobei der Arbeitseinsatz derart ausgestaltet ist, dass der Kontakt der zweiten Stromleitung (50) mit der elektrisch leitenden Schneide (16) in Berührung tritt, wenn der Arbeitseinsatz (29) eingeschoben wird.

2. Medizinisches Instrument nach Anspruch 1, **dadurch gekennzeichnet, dass** die erste Stromleitung (42) isoliert im Inneren des Arbeitseinsatzes (29) zur Schneidekante (37) des Hakens (33) geführt ist.

3. Medizinisches Instrument nach Anspruch 1 oder 2, **dadurch gekennzeichnet, dass** die äußere Seite des Hakens (33) wenigstens teilweise elektrisch isoliert ist.

4. Medizinisches Instrument nach einem der Ansprüche 1 bis 3, **dadurch gekennzeichnet, dass** die zweite Stromleitung (50) isoliert im Inneren des Arbeitseinsatzes (29) zum Kontakt (52) geführt ist.

5. Medizinisches Instrument nach einem der Ansprüche 1 bis 4, **dadurch gekennzeichnet, dass** der Kontakt (52) als Schleifkontakt ausgebildet ist, der beim Hin- und Herbewegen des Arbeitseinsatzes (29) im Außenschaft (12) die Schneide (16) kontaktiert.

6. Medizinisches Instrument nach einem der Ansprüche 1 bis 5, **dadurch gekennzeichnet, dass** die Schneide (16) proximalseitig mit einem umfänglich umlaufenden Abschnitt (65) versehen ist, den der Kontakt (52) kontaktiert.

7. Medizinisches Instrument nach einem der Ansprüche 1 bis 6, **dadurch gekennzeichnet, dass** der Kontakt (52) als seitlich vom Arbeitseinsatz (29) vorspringender Bügel (54) ausgebildet ist.

8. Medizinisches Instrument nach Anspruch 6, wobei der Kontakt (52) als seitlich vom Arbeitseinsatz (29) vorspringender Bögel (54) ausgebildet ist, **dadurch gekennzeichnet, dass** der Bügel (54) seitlich so ausgebogen ist, dass er unter einem Anpressdruck über den Abschnitt (65) der Schneide (16) läuft.

9. Medizinisches Instrument nach einem der Ansprüche 1 bis 8, **dadurch gekennzeichnet, dass** die Schneide (16) als vom distalen Ende (14) des Außenschafts (12) vorspringendes Einsatzstück (56) ausgebildet ist.

10. Medizinisches Instrument nach einem der Ansprüche 1 bis 9, **dadurch gekennzeichnet, dass** die Schneide (16) distalseitig eine Schneidekante (39) aufweist, an der die Schneidekante (3 7) des Hakens (33) vorbei bewegbar ist.

11. Medizinisches Instrument nach Anspruch 10, **dadurch gekennzeichnet, dass** die Schneidekante (39) der Schneide (16) unter einem Winkel zur Schneidekante (37) des Hakens (33) verläuft.

12. Medizinisches Instrument nach einem der Ansprüche 1 bis 11, **dadurch gekennzeichnet, dass** die Schneidekante (37) des Hakens (33) proximalseitig am Haken (33) angeordnet ist.

## Claims

1. Medical instrument for bipolar electro surgery,
with an outer shaft (12) having arranged at its distal end (14) an electrically conductive knife (16) which is electrically isolated against the outer shaft (12), and with a working insert (29) housed in the outer shaft (12) and movable to and fro therein, having a hook (33) at its distal end (31) with a knife edge (37) cooperating with the knife (16) of the outer shaft (12) in a cutting manner,
the working insert (29) has a first current line (42) electrically supplying at least the knife edge (37) of the hook (33), and a second current line (50) is present at the working insert (29) which distally ends in an outer contact (52) being in touch with the electrically conductive knife (16) of the outer shaft (12),
**characterized in that**, the working insert (29) can be fully withdrawn from the outer shaft (12) and can again be inserted into the outer shaft (12), and **in that** the current supply of the knife (16) is provided by the movable working insert (29) housed in the outer shaft, wherein the working insert is designed **in that** the contact of the second current line (50) comes into touch with the electrically conductive knife (16) when inserting the working insert (29).

2. Medical instrument of claim 1, **characterized in that** the first current line (42) is guided in an isolated manner within the working insert (29) to the knife edge (37) of the hook (33).

3. Medical instrument of claims 1 or 2, **characterized in that** the outer face of the hook (33) is at least partially isolated electrically.

4. Medical instrument of any one of claims 1 through 3, **characterized in that** the second current line (50) is guided to the contact (52) within the working insert (29) in an isolated manner.

5. Medical instrument of any one of claims 1 through 4, **characterized in that** the contact (52) is designed as a sliding contact which contacts the knife (16) when moving to and fro the working insert (29) within the outer shaft (12)

6. Medical instrument of any one of claims 1 through 5, **characterized in that** the knife (16) is provided at its proximal side with a circumferentially extending section (65) touching the contact (52).

7. Medical instrument of any one of claims 1 through 6, **characterized in that** the contact (52) is designed as a bow (54) projecting laterally from the working insert (29).

8. Medical instrument of claim 6, wherein the contact (52) is designed as an bow (54) projecting laterally from the working insert (29), **characterized in that** the bow (54) is bent laterally in such a manner that it runs under a contact pressure over the section (65) of the knife (16).

9. Medical instrument of any one of claims 1 through 8, **characterized in that** the knife (16) is designed as an insert (56) projecting from the distal end (14) of the outer shaft (12).

10. Medical instrument of any one of claims 1 through 9, **characterized in that** the knife (16) has at its distal side a knife edge (39) along which the knife edge (37) of the hook (33) can be moved.

11. Medical instrument of claims 10, **characterized in that** the knife edge (39) of the knife (16) runs under an angle to the knife edge (37) of the hook (33).

12. Medical instrument of any one of claims 1 through 11, **characterized in that** the knife edge (37) of the hook (33) is arranged at a proximal side of the hook (33).

## Revendications

1. Instrument médical pour l'électrochirurgie bipolaire,
comprenant une tige extérieure (12), au niveau de l'extrémité distale (14) de laquelle est disposé un tranchant (16) électriquement conducteur, qui est isolé électriquement par rapport à la tige extérieure (12), et
comprenant un insert de travail (29) reçu dans la tige extérieure (12) et déplaçable en va-et-vient axialement dans celle-ci, à l'extrémité distale duquel (31) est disposé un crochet (33) qui présente une arête de coupe (37), qui coopère en coupant avec le tranchant (16) de la tige extérieure (12),
au niveau de l'insert de travail (29) étant prévue une première ligne électrique (42), qui alimente électriquement au moins l'arête de coupe (37) du crochet (33), et
au niveau de l'insert de travail (29) étant prévue une deuxième ligne électrique (50) qui débouche distalement dans un contact extérieur (52) qui est en liaison avec le tranchant électriquement conducteur (16) de la tige extérieure (12),
**caractérisé en ce que** l'insert de travail peut être retiré complètement de la tige extérieure (12) et peut être enfoncé à nouveau dans la tige extérieure (12) et **en ce que** l'alimentation électrique du tranchant (16) s'effectue par l'insert de travail déplaçable (29) reçu dans la tige extérieure, l'insert de travail étant configuré de telle sorte que le contact de la deuxième ligne électrique (50) vienne en contact avec le tranchant électriquement conducteur (16) lorsque l'insert de travail (29) est enfoncé.

2. Instrument médical selon la revendication 1, **caractérisé en ce que** la première ligne électrique (42) est guidée de manière isolée à l'intérieur de l'insert de travail (29) jusqu'à l'arête de coupe (37) du crochet (33).

3. Instrument médical selon la revendication 1 ou 2, **caractérisé en ce que** le côté extérieur du crochet (33) est au moins en partie isolé électriquement.

4. Instrument médical selon l'une quelconque des revendications 1 à 3, **caractérisé en ce que** la deuxième ligne électrique (50) est guidée de manière isolée à l'intérieur de l'insert de travail (29) jusqu'au contact (52).

5. Instrument médical selon l'une quelconque des revendications 1 à 4, **caractérisé en ce que** le contact (52) est réalisé sous forme de contact frottant, qui vient en contact avec le tranchant (16) lors du déplacement en va-et-vient de l'insert de travail (29) dans la tige extérieure (12).

6. Instrument médical selon l'une quelconque des revendications 1 à 5, **caractérisé en ce que** le tranchant (16) est muni du côté proximal d'une portion (65) s'étendant sur tout le pourtour, qui vient en contact avec le contact (52).

7. Instrument médical selon l'une quelconque des revendications 1 à 6, **caractérisé en ce que** le contact (52) est réalisé sous forme d'étrier (54) faisant saillie latéralement depuis l'insert de travail (29).

8. Instrument médical selon la revendication 6, dans lequel le contact (52) est réalisé sous forme d'étrier (54) faisant saillie latéralement depuis l'insert de travail (29), **caractérisé en ce que** l'étrier (54) est cintré latéralement de telle sorte qu'il passe par-dessus la portion (65) du tranchant (16) sous l'effet d'une pression de pressage.

9. Instrument médical selon l'une quelconque des revendications 1 à 8, **caractérisé en ce que** le tranchant (16) est réalisé sous forme de pièce d'insertion (56) faisant saillie depuis l'extrémité distale (14) de la tige extérieure (12).

10. Instrument médical selon l'une quelconque des revendications 1 à 9, **caractérisé en ce que** le tranchant (16) présente du côté distal une arête de coupe (39) devant laquelle l'arête de coupe (37) du crochet (33) peut être déplacée.

11. Instrument médical selon la revendication 10, **caractérisé en ce que** l'arête de coupe (39) du tranchant (16) s'étend suivant un angle par rapport à l'arête de coupe (37) du crochet (33).

12. Instrument médical selon l'une quelconque des revendications 1 à 11, **caractérisé en ce que** l'arête de coupe (37) du crochet (33) est disposée du côté proximal sur le crochet (33).
